# DEMANDE DE BREVET EUROPEEN

(11) **EP 1 759 717 A1**
(43) Date de publication de la demande: **07.03.2007**
(21) Numéro de dépôt: 06119092.2
(22) Date de dépôt: 17.08.2006
(51) Int. Cl.: A61L 9/12

(54) **Capuchon pour un conteneur contenant un agent volatil de traitement d'air**

(30) Priorité: 31.08.2005 FR 0508899
(71) Demandeur: Valeo Systèmes Thermiques, 78321 Le Mesnil St Denis Cedex (FR)
(72) Inventeur: Elliot, Gilles, 91080, Courcouronnes (FR); Cornu, Laurence, 78730, St Arnoult en Yvelines (FR)

(57) **Abrégé**

La présente invention se rapporte à un dispositif de traitement de l'air utilisant un agent traitant volatil contenu à l'intérieur d'un conteneur perméable (1) et un capuchon (4) fermant le conteneur (1). Le capuchon (4) comporte un réservoir (12) renfermant l'agent actif.

## Description

La présente invention concerne un dispositif de traitement de l'air utilisant un agent traitant volatil logé à l'intérieur d'un conteneur perméable et plus particulièrement le capuchon d'un tel dispositif.

De tels dispositifs sont utilisés pour assurer la désinfection, la stérilisation et/ou l'odorisation de l'air,notamment dans les installations de chauffage, ventilation, et/ou climatisation, pour habitacle de véhicule plus particulièrement. Elle a pour objet un capuchon pour un conteneur contenant un agent volatil de traitement d'air notamment pour traiter l'air circulant à travers une telle installation.

On connaît des dispositifs pour traiter l'air utilisant un agent traitant volatil placé à l'intérieur d'un conteneur perméable, pour une diffusion progressive de l'agent traitant à travers la paroi du conteneur perméable sur une période donnée. Ce traitement est par exemple un traitement fongicide, anti-microbien ou odorant.

Selon une première solution connue, l'agent traitant est un liquide contenu à l'intérieur d'un sachet perméable au gaz et imperméable au liquide, lui-même emballé à l'intérieur d'un conteneur imperméable qui est retiré au moment de l'utilisation du dispositif. On pourra par exemple se reporter au document US 4 961 493 qui décrit un tel dispositif.

Inversement et selon une autre solution connue, l'agent traitant est un liquide contenu à l'intérieur d'un sachet imperméable, lui-même contenu dans un conteneur perméable.

Lors de l'utilisation du dispositif, le sachet imperméable est rompu pour libérer l'agent traitant à l'intérieur du conteneur perméable. On pourra par exemple se reporter au document US 5 458 244 qui décrit un tel dispositif.

Néanmoins, le déclenchement du processus de diffusion est difficile car requiert de multiples phases de manipulation. De plus, il est nécessaire de déchirer un emballage enfermant les composants actifs. Cette étape peut engendrer une détérioration du dispositif.

Il est également connu un dispositif de traitement de l'air à partir d'un agent traitant volatil contenu à l'intérieur d'un conteneur perméable, qui offre une diffusion satisfaisante, stable et pérenne de l'agent traitant tout en optimisant la surface disponible de diffusion de l'agent traitant à travers le conteneur perméable afin finalement d'en réduire l'encombrement général.

Toutefois, de tels dispositifs présentent l'inconvénient de ne pas permettre une maîtrise du déclenchement du processus de diffusion. En effet, la diffusion devient opérante dès l'insertion de l'agent traitant dans le conteneur perméable. Ainsi, avant son installation dans l'appareil de traitement de l'air auquel il est destiné, l'agent traitant a commencé à se diffuser au travers des parois du conteneur perméable. La quantité d'agent traitant est donc réduite pour l'utilisation finale au sein d'un appareil de traitement de l'air.

Le but de la présente invention est donc de résoudre les inconvénients décrits précédemment. Elle propose à cet effet un dispositif de traitement de l'air utilisant un agent traitant volatil contenu à l'intérieur d'un conteneur perméable fermé par un capuchon. Selon la présente invention, le capuchon comporte un réservoir renfermant l'agent actif.

Ainsi, une telle réalisation permet de contrôler la phase de déclenchement du processus de traitement de façon simple et rapide puisque celui intervient suite à l'insertion du capuchon dans le conteneur perméable. De plus, la présente invention permet de stocker les produits sans risque de diminution du pouvoir traitant de l'agent traitant.

Selon une première caractéristique additionnelle de l'invention, le réservoir est fermé par un opercule.

Selon une deuxième caractéristique de l'invention, le dispositif comporte des moyens de perforation permettant de percer l'opercule.

Selon un autre mode de réalisation, le dispositif comporte un corps pour le support de l'agent traitant.

Une autre caractéristique de l'invention réside en ce que le conteneur perméable comporte des rainures enserrant le corps.

Alternativement, le capuchon occupe au moins une première et une deuxième position par rapport au conteneur perméable.

Selon une variante de la présente invention, le conteneur perméable comporte une paroi ayant une face intérieure comprenant au moins une gorge d'indexation.

Il est alors couplé, préférentiellement, à un godron disposé sur une paroi extérieure du capuchon et qui coopère avec les gorges d'indexation pour définir diverses positions du capuchon par rapport au conteneur perméable.

Selon un autre mode de réalisation, le conteneur perméable comporte une paroi ayant une face intérieure comprenant un taraudage qui coopère avec un filet réalisé sur une paroi extérieure du capuchon pour réaliser un assemblage par filetage du capuchon dans le conteneur perméable.

Le passage de la première position à la deuxième position se fait par translation ou par rotation du capuchon par rapport au conteneur perméable.

La première position, dite position haute, est telle que l'étanchéité du réservoir est préservée. La deuxième position, dite position basse, est telle que l'étanchéité du réservoir est rompue.

La présente invention est telle que l'étanchéité du réservoir est rompue par les moyens de perforation qui peuvent être le corps comportant un perforateur en sa partie supérieure ou un perforateur additionnel monté dans le conteneur perméable.

Idéalement, le perforateur repose sur les rainures du conteneur perméable.

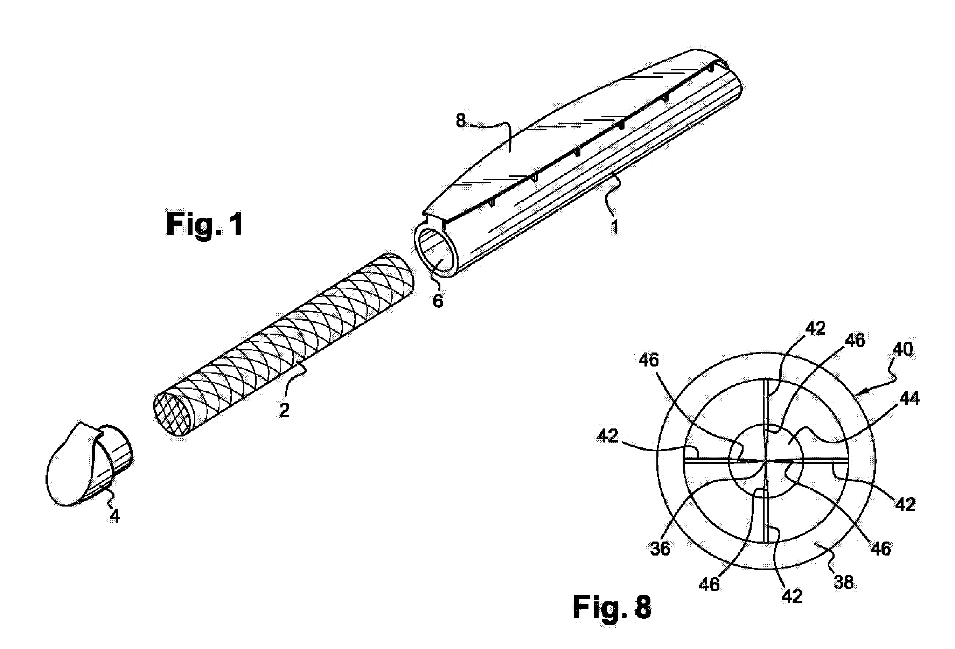

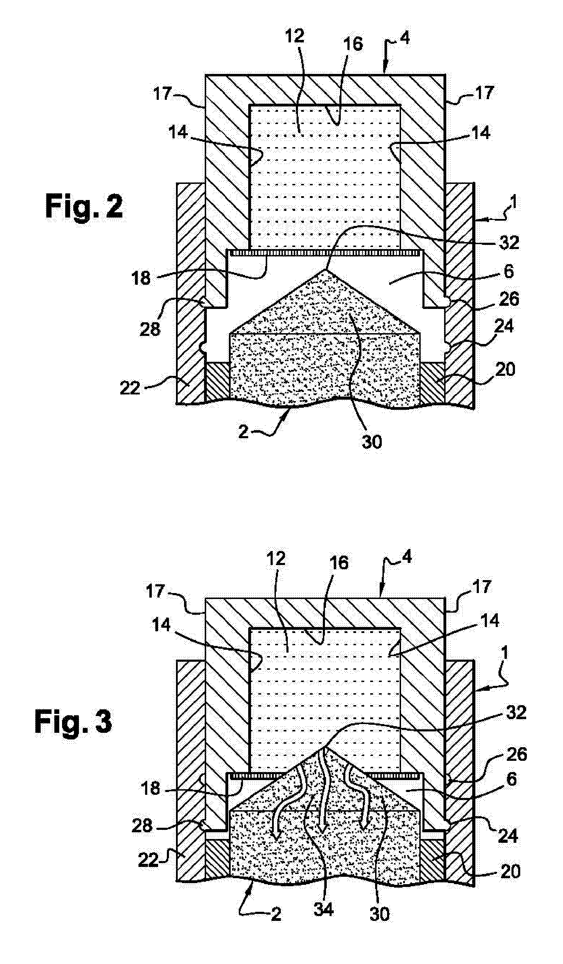

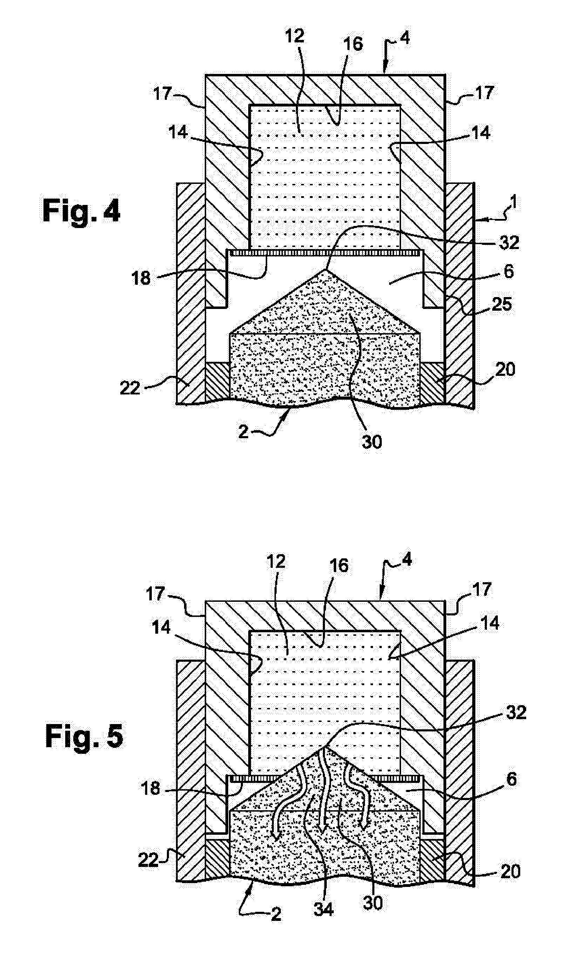

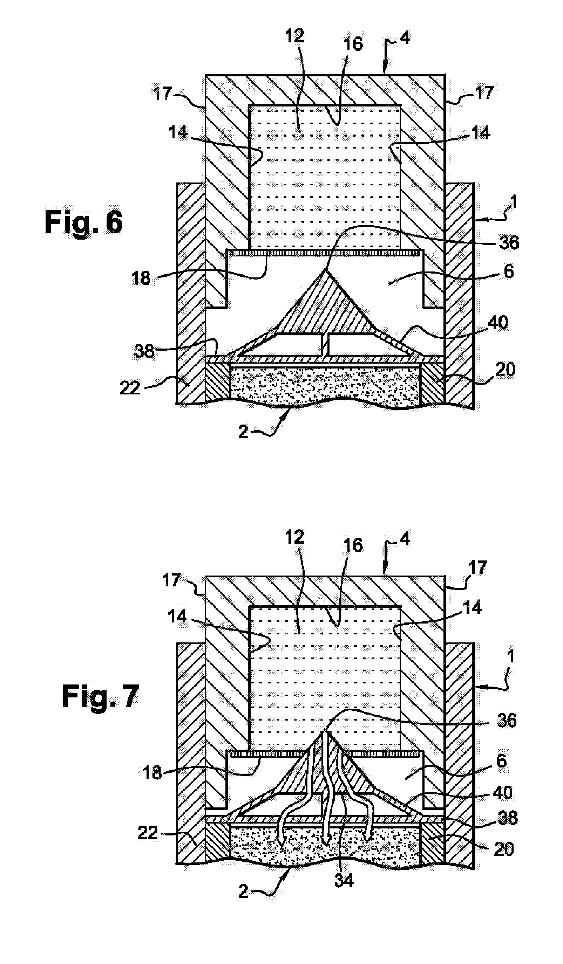

D'autres avantages et caractéristiques de l'invention apparaîtront à la lecture de la description qui suit. Cette description est purement illustrative et est donnée à titre d'exemple et doit être lue en se référant aux dessins annexés sur lesquels on a représenté :
En figure 1, une vue éclatée d'un dispositif de traitement de l'air par un agent traitant selon la présente invention,
En figures 2 et 3, une vue en coupe d'un capuchon de fermeture du dispositif de la présente invention selon un premier mode de réalisation,
En figures 4 et 5, une vue en coupe d'un capuchon de fermeture du dispositif de la présente invention selon un deuxième mode de réalisation,
En figures 6 et 7, une vue en coupe d'un capuchon de fermeture du dispositif de la présente invention selon un troisième mode de réalisation, et
En figure 8, une vue de dessus du perforateur pour un capuchon de fermeture du dispositif de la présente invention selon le troisième mode de réalisation.

On se réfère tout d'abord à la figure 1 qui représente un dispositif de traitement de l'air par un agent traitant.

Le dispositif selon la présente invention est destiné au traitement de l'air à partir d'un agent traitant volatil contenu à l'intérieur d'un conteneur perméable 1.

Selon la présente invention, l'agent traitant est préférentiellement maintenu à l'état liquide, ou de manière analogue, dans un état dans lequel il est susceptible d'être absorbé par une masse spongieuse.

L'agent traitant est conditionné par absorption à l'intérieur d'un corps 2 spongieux servant de support à l'agent traitant logé à l'intérieur du conteneur perméable 1. Le corps 2 présente la caractéristique d'être spongieux en présentant le caractère de pouvoir s'imbiber facilement de l'agent traitant.

Le corps 2 est notamment formé d'une masse poreuse agencée en tampon dont le volume occupe au moins partiellement, par exemple la quasi-totalité, du volume d'une chambre 6 formée par un évidement interne du conteneur perméable 1.

Le corps 2 est sec et est introduit à l'intérieur de la chambre 6 de réception que comporte le conteneur perméable 1. Par la suite, il est imbibé d'un agent traitant à l'état liquide, notamment de l'allyl-isothiocyanate.

La conformation du corps 2 est avantageusement sensiblement complémentaire à celle de la chambre 6, pour optimiser l'occupation du volume utile du conteneur perméable 1 à l'exception d'une mince couche d'air ménagée entre le corps 2 et la paroi du conteneur perméable 1. A cet effet, des rainures 8 assurant le centrage sont disposées sur la paroi intérieure du conteneur perméable 1. Les rainures 20 sont agencées sur une faible portion angulaire. Ainsi, cette disposition permet de restreindre l'encombrement extérieur global du conteneur perméable 1. Grâce à ces dispositions, et au caractère rigide du conteneur perméable 1, l'implantation de ce dernier est facilitée. Sur l'exemple de réalisation illustré, le corps 2 est conformé en tampon cylindrique. Par exemple, il peut être formé à partir d'un feutre textile.

Le volume de la chambre 6 est sensiblement équivalent au volume du corps 2. Toutefois, un espace résiduel entre le corps 2 et la paroi interne du conteneur perméable 1 est créé par les rainures 20 afin de disposer d'une faible couche d'air pour le processus de diffusion.
0,5 mm et 2 mm présente un compromis acceptable pour une diffusion optimale. Plus particulièrement, pour une diffusion annuelle de 45 mg/jour d'agent traitant, notamment de l'allyl-isothiocyanate, à une température constante de l'ordre de 40°C, il est préférable de disposer d'une épaisseur du conteneur perméable 1 de l'ordre de 0,9 mm plus ou moins 20 % pour une surface globale de 4200 mm² plus ou moins 10 %.

Toutefois, ces dimensions sont dépendantes du milieu extérieur environnant, plus particulièrement elles sont fonction de la température, et de l'intensité de la diffusion requise.

Le conteneur perméable 1 est équipé d'un capuchon 4 d'obturation de la chambre 6. Selon la présente invention, le capuchon 4 comporte un réservoir 12 contenant l'agent traitant. Dans une disposition initiale, l'agent traitant est stocké dans le capuchon 4 et est séparé du corps 2.

Le conteneur perméable 1, quant à lui, est rigide et est formé à partir d'une matière plastique perméable, par exemple en polypropylène chargée de talc à 20 %.

Selon une variante de réalisation, le conteneur perméable 1 comporte une excroissance 8 qui permet de fixer le dispositif de traitement d'air sur le média filtrant ou sur le bord périphérique au moyen d'une pince ou d'un clip approprié. Il est également possible de fixer le conteneur perméable 1 sur l'appareil de chauffage, ventilation et/ou climatisation.

La figure 2 présente un premier mode de réalisation selon la présente invention. Selon cet agencement, le capuchon 4 comporte un réservoir 12 contenant l'agent traitant. Le réservoir 12 renferme une quantité d'agent traitant suffisant à l'imprégnation du corps 2 pour un fonctionnement optimal du dispositif de traitement de l'air.

Ce réservoir 12 est réalisé par la paroi intérieure 14 et le fond 16 du capuchon 4. Il définit ainsi un volume compris entre 2 et 10 millilitres. Préférentiellement, il dispose d'un volume de 4,4 millilitres. Toutefois, ces volumes sont susceptibles d'être modulés afin de correspondre aux besoins de traitement du flux d'air, notamment de la durée de vie souhaitée du dispositif de traitement et du climat environnant le conteneur perméable 1.

De façon préférentielle, la paroi intérieure 14 est cylindrique. Toutefois, il est envisageable de prévoir un réservoir ayant une géométrie interne différente.

Le capuchon 4 est en plastique, notamment en polypropylène. Toutefois, les épaisseurs des parois 14 et 16 sont définies de telle sorte que le capuchon 4 ne permette pas la migration de l'agent traitant vers l'extérieur du capuchon 4.

Le réservoir 12 est rendu étanche au milieu extérieur par un opercule 18. De façon similaire, l'opercule 18 assure une parfaite étanchéité entre l'intérieur du réservoir 12 et le milieu extérieur. Ainsi, on s'assure que l'agent traitant ne puisse pas migrer vers l'extérieur du capuchon 4.

Ainsi réalisé, le capuchon 4 comporte une quantité définie d'agent traitant isolée de façon parfaitement étanche du milieu extérieur. Il est donc possible de stocker ces capuchons 4 pendant une période longue sans que la quantité d'agent traitant diminue à cause d'une migration incontrôlée à travers les parois 14 et 16 ou de l'opercule 18.

De façon préférentielle, l'opercule 18 est une feuille d'aluminium qui est solidarisée, par exemple par collage ou soudage, sur la paroi intérieure 14 du capuchon 4. Toutefois, l'opercule 18 peut être réalisé en un film complexe d'aluminium.

Sur la figure 2, le dispositif est décrit dans une première position. Dans cette configuration, le capuchon 4 est inséré dans le conteneur perméable 1 en position haute.

Le conteneur perméable 1 comporte une paroi latérale 22. Selon l'exemple de réalisation, la paroi 22 est cylindrique. Le diamètre intérieur de la paroi 22 est identique au diamètre extérieur du capuchon 4. Un tel assemblage assure un parfait ajustement entre le capuchon 4 et le conteneur perméable 1. Ainsi, une étanchéité parfaite est réalisée entre le capuchon 4 et le conteneur perméable 1.

La paroi 22 comporte sur sa face intérieure des gorges d'indexation 24 et 26. La gorge 26 est disposée au dessus de la gorge 24. La distance séparant les gorges 24 et 26 définit la mise en place du capuchon 4 respectivement en position haute et en position basse.

Les gorges d'indexation 24 et 26 coopèrent avec un godron 28 disposé sur la paroi extérieure 17 du capuchon 4. En position haute, le godron 28 coopère avec la gorge d'indexation 26. En position basse, le godron 28 coopère avec la gorge d'indexation 24. Le godron 28 sert d'index venant s'insérer dans les gorges d'indexation 24 et 26.

Le mode de réalisation décrit comporte deux gorges d'indexation 24 et 26. Selon les besoins, notamment afin de fournir plusieurs positions intermédiaires entre la position haute et la position basse, il est possible d'avoir un nombre de gorges d'indexation plus important.

De même, afin de simplifier la réalisation, il est envisageable de disposer d'une unique gorge d'indexation.

Dans cette configuration, elle indique soit la position haute soit la position basse.

Des nervures 20 sont également disposées sur la face interne du conteneur perméable 1. Elles sont réparties uniformément sur la périphérie et, idéalement, sur de faibles portions angulaires.

Les nervures 20 assurent le centrage du corps 2 afin que celui-ci soit parfaitement centré dans le conteneur perméable 1. De plus, cette disposition, par la répartition périphérique sur de faibles portions angulaires des nervures 20, vise à faciliter l'introduction du corps 2 à l'intérieur de la chambre 6 en ménageant un espace entre le corps 2 et le conteneur perméable 1. Une couche d'air occupe cet espace et favorise le dégagement de l'agent traitant en permettant la diffusion de l'agent traitant.

Selon une alternative, ces nervures 20 sont agencées sur une portion de hauteur du conteneur perméable 1, et préférentiellement en partie supérieure du conteneur perméable 1, afin d'assurer de corps 2 par rapport au capuchon 4 et au réservoir 12.

Le corps 2 est un matériau stabilisant sec placé dans la chambre 6 du conteneur perméable 1. Selon la présente invention, le matériau stabilisant est un corps spongieux, par exemple en feutre. Toutefois, il est envisageable que ce matériau stabilisant soit un matériau minéral, végétal, ou même animal.

Le rôle du corps 2 est, en outre, d'interdire un contact direct entre l'agent traitant, sous sa forme liquide, et la paroi latérale 22 du conteneur perméable 1. Dans le cas inverse, un contact entre l'agent traitant et la paroi latérale 22 aurait des conséquences sur la voie de migration de l'agent traitant à travers la paroi 22, et modifierait donc la vitesse de diffusion de l'agent traitant.

Le corps 2 comporte, en partie supérieure, un perforateur 30. Selon l'exemple de réalisation, le perforateur 30 est directement réalisé en matériau stabilisant. Il prend, idéalement, la forme d'un cône.

Le rôle du perforateur 30 est d'assurer l'ouverture du réservoir 12 et de libérer l'agent traitant lorsque le capuchon 4 est en position basse en perçant l'opercule 18.

Dans l'agencement de la figure 2, la capuchon 4 est mis en place sur le conteneur perméable 1 dans une position haute. Elle correspond notamment à une position de stockage du dispositif de traitement de l'air préalablement à son installation définitive dans un appareil de traitement d'air, comme une installation de chauffage, ventilation et/ou climatisation. Dans cette configuration, le godron 28 coopère avec la gorge d'indexation 26. Le réservoir 12 est hermétiquement clos par l'opercule 18 car le perforateur 30 est distant de l'opercule 18.

La figure 3 présente le dispositif selon le premier mode de réalisation dans une seconde position. Dans cette configuration, le capuchon 4 est inséré dans le conteneur perméable 1 en position basse.

La position basse est la configuration permettant le déclenchement du processus de diffusion de l'agent traitant par l'ouverture de l'opercule 18.

Par rapport à la position haute décrite à la figure 2, le capuchon a été enfoncé, notamment pas une translation du capuchon 4 selon l'axe du conteneur perméable 1, afin que le godron 28 vienne en coopération avec la gorge d'indexation 24.

Les figures 2 et 3 présentent un même dispositif dans deux positions différentes. En conséquence, les éléments communs aux deux figures ont des références identiques à celles décrites en relation avec la figure 2.

Dans cette phase de transition entre la position haute et la position basse, la distance séparant le sommet 32 du perforateur 30 et l'opercule 18 se réduit progressivement. Lorsque le sommet 32 du perforateur 30 entre en contact avec l'opercule 18, il le déchire en créant une ouverture 34.

L'agent traitant présent dans le réservoir 12 clos par l'opercule 18 est libéré. Il imprègne alors le corps 2 par capillarité et se diffuse dans le matériau stabilisant. Ainsi, la totalité du volume du corps 2 contient l'agent traitant.

Dans cette position, l'ouverture 34 est maximale. La diffusion de l'agent traitant est alors optimale.

Le processus de diffusion est alors enclenché et l'agent traitant migre par voie gazeuse à travers la paroi latérale 22 du conteneur perméable 1 afin d'être libéré dans le milieu extérieur.

Selon la présente invention, le perforateur 30 est réalisé par le matériau stabilisant du corps 2. Celui-ci est dimensionné pour assurer une perforation de l'opercule 18. Pour ce faire, les dimensions, forme, dureté, et rigidité sont choisies pour percer l'opercule 18 dans des conditions connues de pression sur le capuchon 4.

Dans l'exemple de réalisation, le perforateur 30 est de forme conique. Néanmoins, il est envisageable de lui donner également une forme pyramidale, une forme cylindrique tronquée suivant un plan incliné ou un cône décentré. De plus, suivant la pression exercée par le perforateur 30 sur l'opercule 18, il est également envisageable d'utiliser un cylindre de faible diamètre ou un parallélépipède de faible section.

On se réfère dorénavant à la figure 4 qui représente un deuxième mode de réalisation du dispositif. L'exemple de réalisation de la figure 4 présente de nombreuses similarités avec les modes de réalisation décrits précédemment. Les éléments communs aux deux exemples sont repris avec des références similaires.

Le dispositif est ici décrit dans une première position. Dans cette configuration, le capuchon 4 est inséré dans le conteneur perméable 1 en position haute.

La réalisation de la figure 4 diffère de celle décrite en figure 2 par le mode de mise en mouvement du capuchon 4. Le mécanisme d'indexation comprenant un godron 28 et des gorges d'indexation 24 et 26 est remplacé par un filetage 25.

Les figures 4 et 5 présentent un même dispositif que celui décrit aux figures 2 et 3 avec un moyen d'assemblage différent. En conséquence, les éléments communs aux figures ont des références identiques à celles décrites en relation avec les figures 2 et 3.

La paroi extérieure 17 du capuchon 4 est filetée alors que la face intérieure de la paroi 22 est taraudée. Ainsi, la mise en place du capuchon 4 dans le conteneur perméable 1 se fait par un montage de type vis-écrou.

La position haute est alors définie par un nombre déterminé de tours du capuchon 4 afin d'assurer la prise de l'assemblage sur un nombre choisi de pas de filet.

La position basse est définie de façon identique par un nombre déterminé de tours du capuchon 4. Toutefois, il peut être également prévu que la position basse est atteinte lorsque le filetage arrive en butée dans le taraudage.

L'assemblage par filetage offre l'avantage de pouvoir plus précisément définir la position du capuchon 4 par rapport au perforateur 30. Ainsi, il est possible de contrôler le débit de l'agent traitant dans le corps 2. En effet, suivant le déplacement du capuchon 4, le perforateur 30 vient percer l'opercule et crée une ouverture 34. L'agent traitant se diffuse par l'ouverture 34 dans le matériau stabilisant du corps 2. Par la forme du perforateur 30 et l'importance de la pénétration du sommet 32 du perforateur 30, il est possible d'avoir une ouverture 34 plus ou mois grande assurant une diffusion plus ou moins importante de l'agent traitant dans le matériau stabilisant du corps 2.

La figure 5 présente le dispositif selon le deuxième mode de réalisation dans une seconde position. Dans cette configuration, le capuchon 4 est inséré dans le conteneur perméable 1 en position basse.

Dans cette position, l'ouverture 34 est maximale. La diffusion de l'agent traitant est alors optimale.

Dans la phase de transition entre la position haute et la position basse, la distance séparant le sommet 32 du perforateur 30 et l'opercule 18 se réduit progressivement. Lorsque le sommet 32 du perforateur 30 entre en contact avec l'opercule 18, il le déchire en créant un ouverture 34.

L'agent traitant présent dans le réservoir 12 clos par l'opercule 18 est libéré. Il imprègne alors le corps 2 par capillarité et se diffuse dans le matériau stabilisant. Ainsi, la totalité du volume du corps 2 contient l'agent traitant.

Le processus de diffusion est alors enclenché et l'agent traitant migre par voie gazeuse à travers la paroi intérieure 22 du capuchon 4 afin d'être libéré dans le milieu extérieur.

Selon la présente invention, le perforateur 30 est réalisé par le matériau stabilisant du corps 2. Celui-ci est dimensionné pour assurer une perforation de l'opercule 18. Pour ce faire, les dimensions, forme, dureté, et rigidité sont choisies pour percer l'opercule 18 dans des conditions connues de pression sur le capuchon 4.

Dans l'exemple de réalisation, le perforateur 30 est de forme conique. Néanmoins, il est envisageable de lui donner également une forme pyramidale, une forme cylindrique tronquée suivant un plan incliné ou un cône décentré. De plus, suivant la pression exercée par le perforateur 30 sur l'opercule 18, il est également envisageable d'utiliser un cylindre de faible diamètre ou un parallélépipède de faible section.

Les figures 6 et 7 représentent un troisième mode de réalisation du dispositif respectivement en position haute et en position basse. La variante de réalisation des figures 6 et 7 présente un dispositif très similaire à celui décrit aux figures 2 et 3 avec un moyen de perforation différent. En conséquence, les éléments communs aux figures ont des références identiques à celles décrites en relation avec les figures 2 et 3.

Le déplacement du capuchon 4 dans ce troisième mode de réalisation peut indifféremment être réalisé par un dispositif d'indexation comme décrit aux figures 2 et 3 ou par un dispositif de filetage tel que décrit aux figures 4 et 5.

Le mode de réalisation de la figure 6 diffère des modes de réalisation précédents par la suppression de la partie supérieure du corps 2 ayant la fonction de perforer l'opercule 18.

En effet, selon le matériau stabilisant utilisé, il est possible que la rigidité du perforateur 30 ne soit pas suffisante pour permettre la pénétration de ce dernier dans l'opercule 18 et de percer ce dernier.

La variante de réalisation de la figure 6 et 7 présente un agencement dans lequel le perforateur 40 est une pièce additionnelle disposée à l'intérieur du conteneur perméable 1.

Le perforateur 40 est, de façon préférentielle, en métal ce qui lui confère la rigidité suffisante pour assurer la perforation de l'opercule 18.

Comme présenté en figure 8, le perforateur 40 comporte une couronne 38 définissant une base à partir de laquelle s'élèvent des arcs 42. Les arcs 42 soutiennent la tête de perforation 44 ayant un sommet 36.

La tête de perforation 44 dispose également de nervures 46. Les nervures 46 servent à créer des arêtes sur la surface lisse de la tête de perforation 44. Elles ont pour rôle de faciliter la séparation de l'opercule 18 et de la tête de perforation 44 afin que l'agent traitant s'écoule facilement vers le matériau stabilisant du corps 2.

Comme présenté en figures 6 et 7, la base 38 du perforateur 40 repose sur les nervures de centrage 20 du conteneur perméable 1. Le diamètre extérieur de la base 38 est légèrement inférieur au diamètre intérieur du conteneur perméable 1. Cela permet d'insérer avec facilité le perforateur dans le conteneur perméable 1.

Le processus de déclenchement de la diffusion de l'agent traitant est identique à celui décrit en relation avec les figures 2 à 5.

Le mode de réalisation des figures 6 et 7 assure une standardisation étendue des dispositifs de traitement d'air par agent traitant. En effet, de tels dispositifs peuvent comporter un corps 2 pré-imprégné d'agent traitant qui est inséré dans le conteneur perméable 1 ou alors comporter, comme la présente invention, un corps 2 'sec' d'agent traitant et un capuchon 4 contenant l'agent traitant.

Quelles que soient les méthodes considérées, il est possible de remplacer et/ou recharger le dispositif par celui décrit ci-dessus.

En effet, si le dispositif que l'on souhaite recharger comportait un corps 2 pré-imprégné d'agent traitant, il est possible de conserver le corps 2 actuellement présent et de remplacer le capuchon initial par un capuchon selon la présente invention et d'insérer un perforateur additionnel comme le perforateur 40 afin de rendre le dispositif de nouveau opérant.

Si le dispositif que l'on souhaite recharger comportait un corps 2 'sec' d'agent traitant et un capuchon à réservoir du type du capuchon 4, il est possible de conserver le corps 2 actuellement présent et de remplacer le capuchon vide d'agent traitant par un nouveau capuchon selon la présente invention comportant une quantité d'agent traitant assurant la prolongation du processus de traitement afin de rendre le dispositif de nouveau opérant.

Selon les modes de réalisation de la présente invention, les moyens de perforation sont constitués soit par une géométrie particulière du corps en matériau stabilisant, soit par une pièce additionnelle venant s'intercaler entre le corps en matériau stabilisant et le capuchon.

Les divers exemples décrits permettent un assemblage entre le capuchon 4 et le conteneur perméable 1. Ce montage est réalisé de façon à assurer un parfait ajustement entre le capuchon 4 et le conteneur perméable 1. Ainsi, une étanchéité parfaite est réalisée entre ces deux éléments. Lors de l'ensemble des étapes d'assemblage du capuchon 4 avec le conteneur perméable 1, l'agent traitant, notamment à l'état liquide, est isolé du milieu extérieur.

En effet, avant la perforation de l'opercule 18 par les moyens de perforation 30 ou 40, l'agent traitant sous forme liquide est dans le réservoir 12 isolé du milieu extérieur par la combinaison de l'opercule 18 et de la paroi intérieure 14 et du fond 16 dont les épaisseurs sont définies de telle façon qu'elles empêchent la migration de l'agent traitant vers l'extérieur du réservoir.

Après perforation de l'opercule 18 par les moyens de perforation 30 ou 40, l'agent traitant est diffusé sous forme liquide et imbibe le corps 2. Le parfait ajustement entre le capuchon 4 et le conteneur perméable assure une étanchéité parfaite entre ces éléments et constitue une garantie d'isolement de l'agent traitant, notamment sous forme liquide, avec le milieu extérieur.

Toutefois, l'Homme de l'Art pourra concevoir les moyens de perforation par tout autre moyen qui permettent de percer l'opercule fermant le réservoir du capuchon.

Un tel dispositif est notamment destiné à être appliqué à une installation de chauffage, ventilation et/ou climatisation, notamment pour véhicule automobile, prenant en compte les contraintes d'utilisation et d'installation du dispositif à l'intérieur d'une telle installation, notamment au regard de l'encombrement du dispositif souhaité le plus faible possible, de la fiabilité, de la pérennité et de la stabilité de la diffusion de l'agent traitant, des modalités aisées d'installation du dispositif à l'intérieur de l'installation ainsi que des modalités de renouvellement du dispositif dans le cas où celui-ci est consommable, voire de l'agent traitant à l'intérieur du dispositif dans le cas contraire.

Le dispositif de traitement de l'air selon la présente invention est propre à être installé à l'intérieur d'un appareil de chauffage, ventilation et/ou climatisation. Notamment, il peut être fixé par exemple sur le média d'un filtre habitacle, du type à particules, à charbon actif ou une combinaison des deux, d'un véhicule automobile de sorte à être disposé dans le circuit d'air qui circule au travers de l'appareil de chauffage, ventilation et/ou climatisation et plus particulièrement de l'évaporateur et du filtre. Ce dernier est constitué d'un bord périphérique ou cadre rigide ou déformable qui maintient le média filtrant sous une forme plissée.

Le dispositif de traitement de l'air peut également être fixé sur le bord périphérique du filtre.

Ces positionnements du dispositif de traitement de l'air sur un filtre lui-même installé à l'intérieur d'une installation de ventilation, chauffage et/ou climatisation permettent de détruire les microbes et odeurs qui peuvent naître à l'intérieur de cette installation.

Dans une phase de stockage, la présente invention assure ainsi un maintien étanche de l'agent traitant. De plus, le corps servant de support à l'agent traitant lors de la diffusion n'est pas imprégné. Ceci permet de conserver sur une période de temps plus importante le pouvoir actif du dispositif.

Le dispositif selon l'invention est propre à être remplacé à chaque changement du filtre habitacle du véhicule ou être réutilisé en le replaçant sur le média du filtre neuf ou sur une paroi de l'installation. Ce dispositif de traitement est ainsi particulièrement adapté à une utilisation dans les réseaux de réparation automobile des constructeurs ou dans les réseaux après-vente indépendants.

La présente invention trouve également une application dans tous les agencements faisant intervenir des flux d'air dont les parois et les composants sont à traiter.

Bien évidemment, l'invention n'est pas limitée aux modes de réalisation décrits précédemment et fournis uniquement à titre d'exemple et englobe d'autres variantes de réalisation que pourra envisager l'homme du métier dans le cadre des revendications.

## Revendications

1. Capuchon (4) pour un conteneur (1) comportant une paroi intérieure (14) et un fond (16) **caractérisé en ce que** la paroi intérieure (14) et le fond (16) forme un réservoir (12).

2. Capuchon (4) selon la revendication 1, **caractérisé en ce que** la paroi intérieure (14) est cylindrique.

3. Capuchon (4) selon la revendication 1 ou 2, **caractérisé en ce que** le réservoir (12) est rendu étanche au milieu extérieur par un opercule (18).

4. Capuchon (4) selon l'une des revendications 1 à 3 2, **caractérisé en ce que** le réservoir (12) contient un agent traitant.

5. Capuchon (4) selon la revendication 4, **caractérisé en ce que** la paroi intérieure (14) et le fond (16) ont des épaisseurs définies afin d'empêcher la migration de l'agent traitant vers l'extérieur du réservoir.

6. Dispositif de traitement de l'air utilisant un agent traitant volatil contenu à l'intérieur d'un conteneur perméable (1) et un capuchon (4) fermant le conteneur (1)
**caractérisé en ce que** le dispositif comporte un capuchon (4) selon l'une des revendications précédentes.

7. Dispositif de traitement de l'air selon la revendication 6, **caractérisé en ce que** le réservoir (12) est fermé par un opercule (18).

8. Dispositif de traitement de l'air selon la revendication 7, **caractérisé en ce que** le dispositif comporte des moyens de perforation (30, 40) propres à percer l'opercule (18).

9. Dispositif de traitement de l'air selon l'une des revendications 6 à 8, **caractérisé en ce que** le dispositif comporte un corps (2) pour le support de l'agent traitant.

10. Dispositif de traitement de l'air selon la revendication 9, **caractérisé en ce que** le conteneur perméable (1) comporte des nervures (20) enserrant le corps (2).

11. Dispositif de traitement de l'air selon l'une des revendications 6 à 10, **caractérisé en ce que** le capuchon (4) occupe au moins une première et une deuxième position par rapport au conteneur perméable (1).

12. Dispositif de traitement de l'air selon l'une des revendications 6 à 11, **caractérisé en ce que** le conteneur perméable (1) comporte une paroi (22) ayant une face intérieure comprenant au moins une gorge d'indexation (24, 26).

13. Dispositif de traitement de l'air selon la revendication 12, **caractérisé en ce que** le capuchon (4) comporte une paroi extérieure (17) comprenant un godron (28) coopérant avec la gorge d'indexation (24, 26) pour définir diverses positions du capuchon (4) par rapport au conteneur perméable (1).

14. Dispositif de traitement de l'air selon l'une des revendications 6 à 11, **caractérisé en ce que** le conteneur perméable (1) comporte une paroi (22) ayant une face intérieure comprenant un taraudage coopérant avec un filet réalisé sur une paroi extérieure (17) du capuchon (4) pour réaliser un assemblage par filetage du capuchon (4) dans le conteneur perméable (1).

15. Dispositif de traitement de l'air selon l'une des revendications 11 à 13, **caractérisé en ce que** le passage de la première position à la deuxième position se fait par translation du capuchon (4) par rapport au conteneur perméable (1).

16. Dispositif de traitement de l'air selon la revendication 14, **caractérisé en ce que** le passage de la première position à la deuxième position se fait par rotation du capuchon (4) par rapport au conteneur perméable (1).

17. Dispositif de traitement de l'air selon l'une des revendications 11 à 16, **caractérisé en ce que** la première position, dite position haute, est telle que l'étanchéité du réservoir (12) est préservée et **en ce que** la deuxième position, dite position basse, est telle que l'étanchéité du réservoir (12) est rompue.

18. Dispositif de traitement de l'air selon la revendication 17, **caractérisé en ce que** l'étanchéité du réservoir (12) est rompue par des moyens de perforation (30, 40).

19. Dispositif de traitement de l'air selon la revendication 17, **caractérisé en ce que** les moyens de perforation sont réalisés par le corps (2) comportant un perforateur (30) en sa partie supérieure.

20. Dispositif de traitement de l'air selon la revendication 17, **caractérisé en ce que** les moyens de perforation sont réalisés par un perforateur (40) additionnel monté dans le conteneur perméable (1).

21. Dispositif de traitement de l'air selon la revendication 20, **caractérisé en ce que** le perforateur (40) repose sur les nervures (20) du conteneur perméable (1).
